# EUROPEAN PATENT APPLICATION

(11) **EP 4 755 897 A1**
(43) Date of publication of application: **10.06.2026**
(21) Application number: 24849236.5
(22) Date of filing: 01.08.2024
(51) Int. Cl.: C07H 21/00, C07H 1/00, C12N 5/10, C12N 15/11, C12N 15/113

(54) **METHOD FOR PRODUCING OLIGONUCLEOTIDE**

(30) Priority: 02.08.2023 JP 2023126123
(71) Applicant: Nippon Shokubai Co., Ltd., Osaka-shi, Osaka 541-0043 (JP)
(72) Inventor: UMEDA NOMURA, Yumi, Suita-shi, Osaka 564-0034 (JP)
(74) Representative: Hoffmann Eitle
(86) International application number: PCT/JP2024/027473
(87) International publication number: WO 2025/028591

(57) **Abstract**

The present application provides a method for producing an oligonucleotide, the method including: cleaving an oligonucleotide from a conjugate containing the oligonucleotide, a cleavable linker, and a support in a solution containing an alkylamine and an organic solvent; and optionally deprotecting a protecting group of the oligonucleotide, wherein the cleavable linker has a cyclic vicinal diol structure; and the organic solvent is selected from an ether, a cyclic amide, and a cyclic urea. According to the present production method, an oligonucleotide can be cleaved with high purity and yield from a conjugate containing the oligonucleotide, a cleavable linker, and a support by suppressing the generation of cleavable-linker-attached by-products and degradation products.

## Description

### Technical Field

The present invention relates to a method for producing an oligonucleotide.

### Background Art

Various methods are known as methods for producing oligonucleotides. For example, a method is known in which an oligonucleotide prepared on a solid-phase support via a cleavable linker is cleaved from the solid-phase support by hydrolyzing the cleavable linker with aqueous ammonia, a methylamine solution, or the like. As the cleavable linker, a cleavable linker having a cyclic vicinal diol structure, for example, a universal linker, is known.

Regarding the cleavage of an oligonucleotide from a conjugate containing a support and a cleavable linker having a cyclic vicinal diol structure, Patent Literature 1 describes a method of cutting out an oligonucleotide by bringing an aqueous solution containing an alkylamine, a monovalent inorganic salt, and an alcohol into contact with the oligonucleotide. However, as described in Comparative Examples 4 and 5 of the present specification, the method of Patent Literature 1 generates cleavable-linker-attached by-products and degradation products, and the purity and yield of the oligonucleotide are not high.

Patent Literature 2 describes the cleavage of an oligonucleotide using a concentrated ammonium hydroxide solution. Patent Literature 3 describes the cleavage of an oligonucleotide using gaseous hydrated ammonia. However, in the method of Patent Literature 2, when the terminal nucleoside of an oligonucleotide attached to a solid-phase support is other than LNA, cleavable-linker-attached by-products and degradation products are generated, and therefore the purity and yield of the oligonucleotide are not high. In the method of Patent Literature 3, a high-temperature high-pressure reaction vessel that can withstand the use of gaseous ammonia is specially required.

### Citation List

### Patent Literature

Patent Literature 1: JP 2017-008038 A
Patent Literature 2: JP 2020-515599 T
Patent Literature 3: JP 2004-513629 T

### Summary of Invention

### Technical Problem

The problem to be solved by the present invention is to provide a method capable of producing an oligonucleotide with high purity and yield by suppressing the generation of cleavable-linker-attached by-products and/or other degradation products in a step of cleaving the oligonucleotide from a conjugate containing the oligonucleotide, the cleavable linker, and a support.

### Solution to Problem

The present inventors have conducted intensive studies in order to solve the problem, and as a result, have found that when an organic solvent selected from an ether, a cyclic amide, and a cyclic urea and/or an organic acid metal salt is used in the cleavage step, unexpectedly, an oligonucleotide can be produced with high purity and yield by suppressing the generation of by-products and/or other degradation products, thereby completing the present invention. That is, the present invention is as follows.
[1] A method for producing an oligonucleotide, the method including:
   cleaving an oligonucleotide from a conjugate containing the oligonucleotide, a cleavable linker, and a support in a solution containing an alkylamine and an organic solvent; and
   optionally deprotecting a protecting group of the oligonucleotide, wherein
   the cleavable linker has a cyclic vicinal diol structure; and
   the organic solvent is selected from an ether, a cyclic amide, and a cyclic urea.
[2] The method according to [1], wherein the solution further contains an organic acid metal salt.
[3] The method according to [2], wherein the organic acid is selected from a substituted or unsubstituted alkylsulfonic acid, a substituted or unsubstituted arylsulfonic acid, a substituted or unsubstituted alkanoic acid, and a substituted or unsubstituted alkanedioic acid.
[4] The method according to [2] or [3], wherein the metal salt is an alkali metal salt.
[5] A method for producing an oligonucleotide, the method including:
   cleaving an oligonucleotide from a conjugate containing the oligonucleotide, a cleavable linker, and a support in a solution containing an alkylamine; and
   optionally deprotecting a protecting group of the oligonucleotide, wherein:
      the cleavable linker has a cyclic vicinal diol structure; and
      the solution further contains an organic acid metal salt.
[6] The method according to [5], wherein the organic acid is selected from a substituted or unsubstituted alkylsulfonic acid, a substituted or unsubstituted arylsulfonic acid, a substituted or unsubstituted alkanoic acid, and a substituted or unsubstituted alkanedioic acid.
[7] The method according to [5] or [6], wherein the metal salt is an alkali metal salt.

### Advantageous Effects of Invention

According to the production method of the present invention, an oligonucleotide can be produced with high purity and yield from a conjugate containing the oligonucleotide, a cleavable linker, and a support by suppressing the generation of cleavable-linker-attached by-products and/or other degradation products.

### Description of Embodiments

The method for producing an oligonucleotide of the present invention is a production method for an oligonucleotide, the method including: a step of cleaving an oligonucleotide from a conjugate containing the oligonucleotide, a cleavable linker, and a support in a solution containing an alkylamine and an organic solvent; and a step of optionally deprotecting a protecting group of the oligonucleotide, wherein the cleavable linker has a cyclic vicinal diol structure; and the organic solvent is selected from an ether, a cyclic amide, and a cyclic urea.

In the production method of the present invention, the step of cleaving an oligonucleotide from the conjugate and the step of deprotecting a protecting group of the oligonucleotide may be performed in the same step, or the latter step may be performed after the former step, or the former step may be performed after the latter step. The conjugate containing an oligonucleotide, a cleavable linker, and a support according to the present invention particularly refers to an aspect in which the cleavable linker is covalently attached to each of the support and the oligonucleotide.

The "support" is not particularly limited as long as it is a structure capable of anchoring the 3'-end side or 5'-end side of the oligonucleotide to be synthesized via a linker molecule. The support is a solid-phase support or a liquid-phase support, and preferably a solid-phase support. Examples of the solid-phase support that can be used include a porous support such as a glass-based support, a polystyrene-based support, a silica gel-based support, or an acrylamide-based support. The solid-phase support is preferably a glass-based support, a polystyrene-based support, or a silica gel-based support, particularly preferably a glass-based support or a polystyrene-based support. The acrylamide-based support refers to a porous support formed of a resin mainly composed of a structural unit of acrylamide or a derivative thereof. The polystyrene-based support refers to a porous support formed of a resin mainly composed of a structural unit of styrene or a derivative thereof.

The support has a functional group for attaching to a cleavable linker, such as an amino group or a hydroxyl group. Specific examples of the solid-phase support include those described in JP 2011-088843 A and JP 2013-177371 A. In a preferred embodiment, low-swelling polystyrene particles commercially available as NittoPhase (trade name) (available from Kinovate) or Primer Support (trade name) (available from Cytiva) can be used. The average particle diameter of the porous support used for the synthesis of an oligonucleotide is preferably from 1 to 1000 µm, more preferably from 5 to 500 µm, and still more preferably from 10 to 200 µm.

The "cleavable linker having a cyclic vicinal diol structure" is also referred to as a universal linker, and examples thereof include a cleavable linker having bicyclo[2.2.1]heptane in which methylene may be substituted with an oxygen atom, a sulfur atom, or an imino group, and a cleavable linker having bicyclo[3.2.1]octane in which methylene may be substituted with an oxygen atom, a sulfur atom, or an imino group. Specific examples of the cleavable linker having a cyclic vicinal diol structure include Unylinker, cleavable linkers described in Patent Literatures 1 to 3, and cleavable linkers described below. The cleavable linker is preferably selected from any of the following Formulae (A), (B), and (C). The cleavable linker is more preferably a cleavable linker of the following Formula (B), still more preferably a cleavable linker of the following Formula (B) (X = NH or O, R = phenyl).

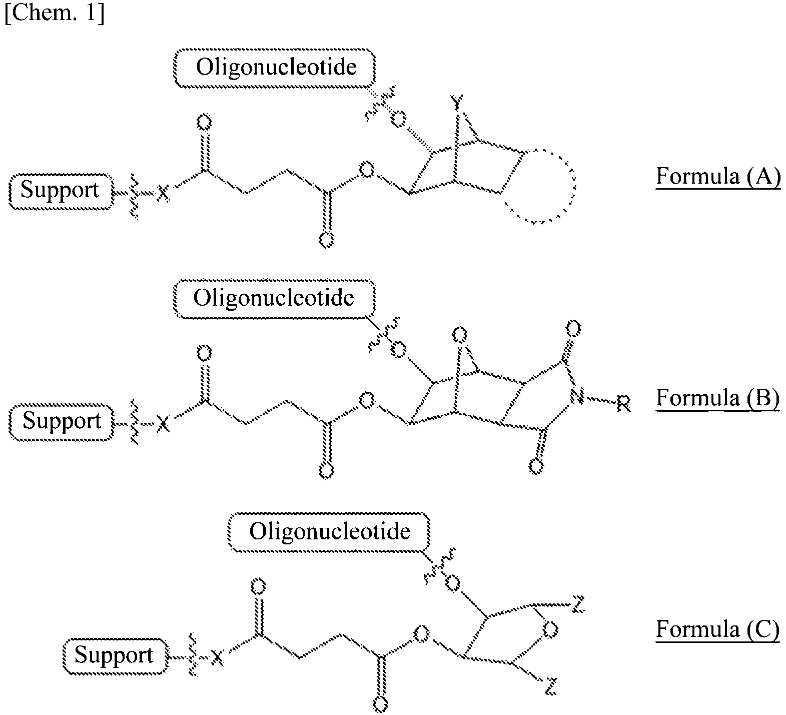

[In the Formulae, X is an oxygen atom or NH.

Y is methylene, an oxygen atom, a sulfur atom, NH, an optionally substituted divalent aromatic hydrocarbon group (for example, optionally substituted phenylene), or the like.

The dashed line denotes a ring that is either absent or condensed to the bicyclo ring.

R is optionally substituted alkyl, optionally substituted aryl, or the like, and preferably methyl, isopropyl, or phenyl.

Z is alkyl, alkoxy, aryl, acyl, or the like, and preferably methoxy.]

One hydroxyl group of the cyclic vicinal diol structure of the cleavable linker is attached to the support via, for example, succinic acid and an ester bond, and the other hydroxyl group is attached to the oligonucleotide via a phosphoester bond.

Examples of the "oligonucleotide" include RNA oligonucleotides, DNA oligonucleotides, and oligonucleotides containing a modified sugar moiety or a modified base, and preferred examples thereof include RNA oligonucleotides and RNA oligonucleotides containing a modified sugar moiety and/or a modified base. The number of bases of the oligonucleotide is, for example, from 2 to 200, preferably from 3 to 150, more preferably from 5 to 100, and still more preferably from 7 to 50. Examples of the modified sugar moiety include those having ribose, hexitol, morpholine, or N-(2-aminoethyl)glycine as a backbone. The modification to ribose may be, for example, modification at the 2'-position or the 5'-position, or bridge-type modification. Specific examples of the modification at the 2'-position include, for example, 2'-F, 2'-O-methyl (2'-OMe), 2'-O-methoxyethyl (2'-MOE), and 2'-arabinosyl-F (2'-FANA). Specific examples of the modification at the 5'-position include, for example, 5'-methyl (5'-Me) and 5'-cyclopropylene (5'-CP). Specific examples of the bridge-type modification include those in which a bridge structure is introduced between the 2'-position and the 4'-position, and examples thereof include 2',4'-BNA (LNA), 2',4'-BNACOC, 2',4'-BNANC, 2'-amino LNA, ENA, AmNA, scpBNA, cEt, and GuNA.

Examples of the modified base include those having a heterocyclic ring as a backbone, and examples of the heterocyclic ring include a purine derivative and a pyrimidine derivative. Examples of the purine derivative include adenine, guanine, isoguanine, xanthine, and hypoxanthine, each of which may have a substituent. Examples of the pyrimidine derivative include cytosine, thymine, and uracil, each of which may have a substituent. Examples of the substituent include a hydroxyl group, an alkyl group, an alkenyl group, an alkynyl group, an allyl group, an aryl group, a fluoro group, a chloro group, a bromo group, an iodo group, and a carboxyl group. Preferred examples of the purine derivative base having a substituent include an 8-bromoadenyl group, an 8-bromoguanyl group, and an 8-oxoguanyl group. Preferred examples of the pyrimidine derivative base having a substituent include a 5-methylcytosyl group (mC), a 5-bromocytosyl group, a 5-bromouracil group, a 5-iodouracil group, a 5-iodocytosyl group, a 5-fluorouracil group, and a 5-(N-aminohexyl-3-acrylimide) group. The oligonucleotide in the present invention has a structure in which, for example, nucleosides are linked by a phosphodiester bond, a phosphorothioate bond, a phosphorodithioate bond, a phosphoramidate bond, or a boranophosphate bond, preferably a phosphodiester bond or a phosphorothioate bond.

The oligonucleotide is produced by sequentially attaching nucleotides starting from the other hydroxyl group of the cleavable linker attached to the support in accordance with a common method. If necessary, the functional group on the base moiety (e.g., amino group), the functional group on the sugar moiety (e.g., hydroxyl group at the 2'-position), and the phosphoester moiety of the nucleotide are protected by protecting groups. As the protecting group for the oligonucleotide, various protecting groups used in the production of oligonucleotides are known in the art, and a suitable protecting group can be appropriately selected from these various protecting groups and used. The protecting group for the functional group on the base moiety can include, for example, an acyl group or a dimethylformamidyl group, and is preferably selected from an isobutyryl group, an acetyl group, a benzoyl group, a phenoxyacetyl group, an isopropylphenoxyacetyl group, a tert-butylphenoxyacetyl group, and a dimethylformamidyl group, and is more preferably a benzoyl group, an acetyl group, or an isobutyryl group. The protecting group for the functional group on the sugar moiety can include, for example, a silyl-based protecting group and an optionally substituted alkyl group, and is preferably a tert-butyldimethylsilyl group, a tert-butyldiphenylsilyl group, or a triisopropylsiloxymethyl group. Examples of the protecting group for the phosphoester moiety include a cyanoethyl group.

The reaction mechanism for cleaving an oligonucleotide is considered to be that, for example, (1) an alkylamine cleaves an ester bond between the support and the cleavable linker to remove the support, and (2) a negative charge generated at the position of one hydroxyl group of the vicinal diol in the cleavable linker causes a nucleophilic substitution reaction to the phosphoester at the position of the other hydroxyl group to form a cyclic phosphodiester containing the cleavable linker, whereby an oligonucleotide from which the phosphate group at the terminal is removed is cleaved.

When step (2) does not proceed and the reaction is terminated in step (1), a by-product in which the cleavable linker is attached to the oligonucleotide is generated, resulting in a decrease in the purity and yield of the oligonucleotide.

When the reaction conditions are made severe so as to promote the cleavage of the phosphoester bond between the hydroxyl group of the cleavable linker and the oligonucleotide in order to suppress the generation of the by-product, the degradation of the oligonucleotide also proceeds to form a degradation product, resulting in a decrease in the purity and yield of the oligonucleotide.

The production method of the present invention can suppress the generation of cleavable-linker-attached by-products and degradation products in the cleavage step, whereby an oligonucleotide can be produced with high purity and yield.

Examples of the "alkylamine" include a monoamine and a diamine. Examples of the monoamine include a primary alkylamine, a secondary alkylamine, or a tertiary alkylamine. The primary amine is, for example, a linear or branched monoalkylamine, preferably has from 1 to 10 carbons in the alkyl moiety, and is more preferably methylamine, ethylamine, n-propylamine, n-butylamine, tert-butylamine, n-pentylamine, or n-hexylamine, still more preferably methylamine, ethylamine, or tert-butylamine. The secondary amine is, for example, a linear or branched dialkylamine, and preferably has from 1 to 10 carbons in the alkyl moiety. More preferred examples thereof include dimethylamine, diethylamine, methylethylamine, di-n-propylamine, din-butylamine, and di-tert-butylamine. The tertiary amine is, for example, a linear or branched trialkylamine, and preferably has from 1 to 10 carbons in the alkyl moiety. More preferred examples thereof include trimethylamine and triethylamine. Preferred monoamine is a primary amine.

The diamine is preferably a linear diamine, more preferably a linear diaminoalkane, still more preferably 1,2-diaminoethane, 1,2-diaminopropane, 1,3-diaminopropane, or 1,4-diaminobutane, and even more preferably ethylenediamine.

The alkylamine may be used, for example, in the form of an aqueous solution. Typically, the alkylamine is preferably used in a large excess amount relative to the conjugate containing the oligonucleotide, the cleavable linker, and the support. For example, the alkylamine is used in an amount of from 5 to 100 equivalents, more preferably from 10 to 50 equivalents, per base length relative to 1 mol of the oligonucleotide.

The reaction temperature and the reaction time are preferably selected as appropriate reaction conditions depending on the oligonucleotide and the cleavable linker. The reaction temperature is selected from a range of, for example, from 10 to 60°C, more preferably from 25 to 50°C, and still more preferably from 30 to 40°C. When the reaction temperature is too high, the degradation of the oligonucleotide proceeds to generate a degradation product, resulting in a decrease in the purity and yield of the oligonucleotide. When the reaction temperature is too low, a long reaction time is required. As for the reaction time, it is preferable to monitor the progress of the reaction and terminate the reaction at an optimal time.

As "ether, cyclic amide, and cyclic urea" used as the organic solvent, it is preferable to use an organic solvent that can dissolve reactants and is not degraded under the reaction conditions, and it is more preferable to use an ether.

Examples of the "ether" include chain ethers and cyclic ethers. Examples of the chain ethers include diethyl ether, diisopropyl ether, dibutyl ether, dibenzyl ether, methyl-tert-butyl ether (MTBE), ethyl-tert-butyl ether (ETBE), methyl-tert-amyl ether (MTAE), ethyl-tert-amyl ether (ETAE), cyclopentyl methyl ether (CPME), dimethoxyethane, and dipropylene glycol dimethyl ether. Preferably, the chain ether is diisopropyl ether, MTBE, ETBE, CPME, or dimethoxyethane, more preferably dimethoxyethane. Examples of the cyclic ethers include tetrahydrofuran (THF), tetrahydropyran, 2-methyltetrahydrofuran, 4-methyltetrahydropyran, 1,4-dioxane, and dihydrolevoglucosenone. Preferably, the cyclic ether is THF or 1, 4-dioxane, more preferably THF. Examples of the "cyclic amide" include those having a five-membered ring or a six-membered ring. Preferably, the cyclic amide is N-methylpyrrolidone. Examples of the "cyclic urea" include those having a five-membered ring or a six-membered ring. Preferably, the cyclic urea is 1,3-dimethyl-2-imidazolidinone or N,N'-dimethylpropyleneurea, more preferably 1,3-dimethyl-2-imidazolidinone.

As can be seen from the comparison between Example 1 and Comparative Examples 3 to 5 described below, the present invention using an organic solvent selected from an ether, a cyclic amide, and a cyclic urea can efficiently cleave an oligonucleotide by suppressing the generation of by-products, degradation products, and the like, and can produce an oligonucleotide with high purity and yield, unlike the invention of Patent Literature 1 using ethanol or isopropanol.

As the "organic acid" of the "organic acid metal salt", it is preferable to use an organic acid that can be dissolved in the reaction solution of the present reaction in a state of the organic acid metal salt and can be separated and removed from the oligonucleotide. Examples of the organic acid include substituted or unsubstituted alkylsulfonic acids, substituted or unsubstituted arylsulfonic acids, substituted or unsubstituted alkanoic acids, substituted or unsubstituted alkanedioic acids, substituted or unsubstituted unsaturated carboxylic acids, and substituted or unsubstituted arylcarboxylic acids. Preferably, the organic acid is selected from alkylsulfonic acids, arylsulfonic acids, and alkanoic acids. More preferably, the organic acid is an arylsulfonic acid or an alkanoic acid. Examples of the substituent used for the substitution as described above include mercapto, amino, hydroxy, alkyl, alkenyl, alkynyl, alkanoyl, aryl, nitro, cyano, fluoro, chloro, bromo, and iodo, and preferably include mercapto, amino, hydroxy, and alkyl. Examples of the alkylsulfonic acid include linear or branched C₁₋₁₀ alkylsulfonic acids and substituted products thereof, and preferably include methanesulfonic acid, ethanesulfonic acid, isethionic acid, taurine, N-methyltaurine, 3-mercapto-1-propanesulfonic acid, and 10-camphorsulfonic acid. Examples of the arylsulfonic acid include benzenesulfonic acid, p-toluenesulfonic acid, and naphthalenesulfonic acid, and preferably include p-toluenesulfonic acid. Examples of the alkanoic acid include linear or branched C₂₋₁₀ alkanoic acids and substituted products thereof, and preferably include acetic acid, propionic acid, butanoic acid, trifluoroacetic acid, glycine, lactic acid, and pyruvic acid. Examples of the alkanedioic acid include linear or branched C₃₋₁₀ alkanedioic acids and substituted products thereof, and preferably include oxalic acid, malonic acid, succinic acid, glutaric acid, and glutamic acid. Examples of the unsaturated carboxylic acid include linear or branched C₃₋₁₀ unsaturated carboxylic acids and substituted products thereof, and preferably include acrylic acid, methacrylic acid, crotonic acid, butenoic acid, pentenoic acid, propiolic acid, and maleic acid. Examples of the arylcarboxylic acid include benzoic acid and phthalic acid, and preferably include benzoic acid.

Examples of the "metal salt" of the "organic acid metal salt" include alkali metal salts (including a lithium salt, a sodium salt, a potassium salt, and a rubidium salt) and alkaline earth metal salts (including a magnesium salt), preferably include alkali metal salts (including a lithium salt, a sodium salt, and a potassium salt), and more preferably include a lithium salt, a sodium salt, and a potassium salt.

The amount of the organic acid metal salt to be added is, for example, an amount that provides a concentration of from 10 to 1000 mM in the reaction mixture, preferably an amount that provides a concentration of from 20 to 500 mM in the reaction mixture, and more preferably an amount that provides a concentration of from 50 to 300 mM in the reaction mixture.

When the organic acid metal salt described above is used, an oligonucleotide can be cleaved with high purity and yield from the conjugate containing the oligonucleotide, the cleavable linker, and the support by suppressing the generation of cleavable-linker-attached by-products and degradation products.

The deprotection conditions for protecting groups of the oligonucleotide are well known in the art for each protecting group. In the production method of the present invention, suitable deprotection conditions can be appropriately selected from these deprotection conditions and used.

Another method for producing an oligonucleotide of the present invention is a production method for an oligonucleotide, the method including a step of cleaving an oligonucleotide from a conjugate containing the oligonucleotide, a cleavable linker, and a support in a solution containing an alkylamine and an organic acid metal salt, and a step of optionally deprotecting a protecting group of the oligonucleotide. As the cleavable linker and the organic acid metal salt, those used in the above-described production method can be suitably used.

The present production method can be carried out in accordance with the description of the aforementioned production method.

### Examples

The present invention will be described in more detail with reference to Examples and the like below, but the present invention is not limited to these Examples and the like.

Unless otherwise specified, in oligonucleotides such as RNA in the Examples and the like, the sugar moiety is a ribose ring, the base moiety is selected from adenine, cytosine, guanine, and uracil, and the bond between nucleotides is a phosphodiester bond. In the Examples, 2'-F (2'-fluoro RNA nucleotide), unless otherwise specified, has a structure in which the sugar moiety is a ribose ring having the 2'-hydroxyl group substituted with a fluorine atom, and the base moiety is selected from adenine, cytosine, guanine, and uracil; 2'-OMe (2'-methoxy RNA nucleotide), unless otherwise specified, has a structure in which the sugar moiety is a ribose ring having the 2'-hydroxyl group substituted with a methoxy group, and the base moiety is selected from adenine, cytosine, guanine, and uracil; 2'-MOE (2'-O-methoxyethyl RNA nucleotide), unless otherwise specified, has a structure in which the sugar moiety is a ribose ring having the 2'-hydroxyl group substituted with a methoxyethoxy group, and the base moiety is selected from adenine, cytosine, guanine, and thymine; and LNA, unless otherwise specified, has a structure in which the sugar moiety is a ribose ring having the 2'-oxygen atom and the 4'-carbon atom bridged by a methylene group, and the base moiety is selected from adenine, 5-methylcytosine, guanine, and thymine.

### Measurement of Amount of Target Oligonucleotide (FLP), By-products, and Impurities

### (Degradation Products)

The crude liquid of each of the oligonucleotides produced in the Examples and the like described below was measured by ultra-high performance liquid chromatography (UHPLC) under the following conditions, and the proportions of the target oligonucleotide (FLP), by-products (Unylinker-attached products), and impurities (degradation products) (target peak area/total area of all peaks (%)) were calculated. The impurities include not only degradation products generated by the cleavage of the oligonucleotide but also degradation products generated during the preparation of a solid-phase carrier to which the oligonucleotide is attached.

Measurement conditions: UHPLC instrument; ACQUITY (trade name), UPLC (trade name), column; Waters ACQUITY UPLC Oligonucleotide BEH C18, 130 Å, 1.7 µm, 2.1 mm × 100 mm, UV detection; 260 nm, Buffer A; 100 mM HFIP, 8 mM TEA in H₂O:MeCN = 98:2, Buffer B; H₂O:MeCN = 50:50, temperature; 60°C

Furthermore, for each sample solution of the crude liquids of the oligonucleotides produced in the Examples and the like, the absorbance was measured with a spectrophotometer, and the yield (the ratio of an OD value obtained by actual measurement to a theoretically obtainable maximum OD value) was calculated from the obtained OD value.

### Example 1

Using a nucleic acid synthesizer OligoPilot (trade name) plus 100, a reaction was performed to synthesize an oligonucleotide (FLP) having a base sequence of SEQ ID NO: 1 by sequentially condensing phosphoramidites to a nucleoside attached to a solid-phase carrier via a linker.

Next, the solid-phase carrier after the above reaction was immersed and shaken at 25°C for 30 minutes in a mixed solution of 40 wt.% aqueous methylamine solution/THF (volume ratio 3:7) having a liquid amount of 100 mL per mmol of the linker loaded on the solid-phase carrier, to thereby cleave the bond between the solid-phase carrier and the cleavable linker.

Subsequently, the solution after the above cleavage was filtered to remove the solid-phase carrier. The solid-phase carrier was then washed with a mixed solution of 40 wt.% aqueous methylamine solution/THF (volume ratio 3:7). To the combined washing and filtrate, sodium acetate was added so as to achieve a concentration of 150 mM. The mixture was then shaken at 35°C for 6 hours to deprotect protecting groups (acetyl group (cytosine), benzoyl group (adenine), and isobutyryl group (guanine)) of the amino group (-NH₂) on the base moieties of the target oligonucleotide and to remove the linker. Further, to this liquid, dimethyl sulfoxide and triethylamine trihydrofluoride were added, and the mixture was shaken at 55°C for 1 hour to deprotect the protecting group of the hydroxyl group at the 2'-position of the target oligonucleotide.

To the liquid after the deprotection, acetonitrile was added to precipitate the target oligonucleotide. The resulting precipitate was then collected and dissolved in water to prepare a crude liquid of the target oligonucleotide.

The solid-phase carrier, the linker, and the target oligonucleotide described above are as follows.
Solid-phase carrier, linker: NittoPhase (trade name) HL Unylinker 250 (Kinovate)
Target oligonucleotide (FLP): 5'-acuguuauacucagaaucgcg-3 ' (SEQ ID NO: 1: 21mer, each nucleoside unit indicated by a lowercase letter represents a structural unit identical to that of RNA.)

### Examples 2 to 4 and Comparative Examples 1 and 2

Crude liquids of oligonucleotides of Examples 2 to 4 and Comparative Examples 1 and 2 were obtained by performing the same operation under the same conditions as in Example 1, except that sodium acetate used in Example 1 was changed to any of the organic acid metal salts shown in Table 1.

The amounts of the target oligonucleotide (FLP) and by-products were measured for the crude liquid of each of the oligonucleotides produced in Examples 1 to 4 and Comparative Examples 1 and 2, and the results are shown in Table 1.

**[Table 1]**

| | Organic acid metal salt | FLP (%) | By-products (%) | Degradation products and the like (%) | Yield (%) |
|---|---|---|---|---|---|
| Example 1 | Sodium acetate | 73 | ≤ 0.2 | 15 | 66 |
| Example 2 | Lithium acetate dihydrate | 72 | ≤ 0.2 | 16 | 59 |
| Example 3 | Potassium acetate | 73 | 1.3 | 14 | 59 |
| Example 4 | Magnesium acetate tetrahydrate | 72 | 1.6 | 17 | 51 |
| Comparative Example 1 | Cesium acetate | 67 | 7.1 | 14 | 51 |
| Comparative Example 2 | Calcium acetate monohydrate | 58 | 0.4 | 12 | 50 |

As shown in Table 1, in Examples 1 to 4, the amount of the by-products (Unylinker-attached products) was not large, the amount of the degradation products was not large, and the target oligonucleotide (FLP) was cleaved. From the results of Examples 1 to 4 and Comparative Examples 1 and 2, it was found that the alkali metal salts (sodium salt, lithium salt, potassium salt) and the magnesium salt are preferable because they can efficiently cleave the oligonucleotides, and that the alkali metal salts (sodium salt, lithium salt, potassium salt) are more preferable because the oligonucleotides can be produced with high purity and yield.

### Examples 5 to 12

To a mixed solution of 40 wt.% aqueous methylamine solution/any of the solvents shown in Table 2 (volume ratio 6:4) having an amount of 100 mL per mmol of the loading on the solid-phase carrier, any of the organic acid metal salts shown in Table 2 was added so as to achieve a concentration of 150 mM. In this solution, the solid-phase carrier to which the target oligonucleotide synthesized in Example 1 was attached (i.e., the solid-phase carrier before the cleavage step and the deprotection step) was immersed and shaken at 45°C for 4 hours to cleave the target oligonucleotide from the solid-phase carrier and to deprotect the protecting groups on the base moieties. Thereafter, the solid-phase carrier was removed by filtration and washed with dimethyl sulfoxide. To the combined washing and filtrate, triethylamine trihydrofluoride was added. The mixture was then shaken at 55°C for 1 hour to deprotect the 2'-hydroxyl groups of the target oligonucleotide. To this liquid, acetonitrile was added to precipitate the target oligonucleotide, and the resulting precipitate was then dissolved in water to prepare a crude liquid of the target oligonucleotide.

The amounts of the target oligonucleotide (FLP) and by-products were measured for the crude liquid of each of the oligonucleotides produced in Examples 5 to 12, and the results are shown in Table 2.

**[Table 2]**

| | Organic acid metal salt | Organic solvent | FLP (%) | By-products (%) | Degradation products and the like (%) | Yield (%) |
|---|---|---|---|---|---|---|
| Example 5 | Sodium acetate | THF | 74 | 0.7 | 15 | 67 |
| Example 6 | Sodium 3-mercapto-1-propanesulfonate | THF | 74 | 0.4 | 15 | 66 |
| Example 7 | Sodium p-toluenesulfonate | THF | 74 | 0.6 | 15 | 64 |
| Example 8 | Sodium isethionate | THF | 73 | 0.7 | 16 | 56 |
| Example 9 | Sodium glutamate | THF | 74 | 0.7 | 15 | 68 |
| Example 10 | Sodium acetate | Dimethoxyethane | 71 | 0.4 | 16 | 49 |
| Example 11 | Sodium p-toluenesulfonate | N-methylpyrrolidone | 73 | 0.7 | 16 | 67 |
| Example 12 | Sodium p-toluenesulfonate | N,N-dimethylimidazolidinone | 73 | 0.8 | 15 | 66 |

As shown in Table 2, in Examples 5 to 12, the amount of by-products (Unylinker-attached products) was small, the amount of degradation products was also small, and the target oligonucleotide (FLP) was cleaved. It was found that all of the organic acid salts used in Examples 5 to 12 are preferable. It was found that all of the organic solvents used in Examples 5 to 12 are preferable, and that THF, N-methylpyrrolidone, and N, N-dimethylimidazolidinone are more preferable.

### Examples 13 to 16

To a mixed solution of 40 wt.% aqueous methylamine solution/THF having the liquid amount and THF vol.% shown in Table 3 per mmol of the loading on the solid-phase carrier, sodium 3-mercapto-1-propanesulfonate was added so as to achieve the salt concentration shown in Table 3. In this solution, the solid-phase carrier to which the target oligonucleotide synthesized in Example 1 was attached (i.e., the solid-phase carrier before the cleavage step and the deprotection step) was immersed and shaken at the temperature and for the time shown in Table 3 to cleave the target oligonucleotide from the solid-phase carrier and to deprotect the protecting groups on the base moieties. Thereafter, the solid-phase carrier was removed by filtration and washed with dimethyl sulfoxide. To the combined washing and filtrate, triethylamine trihydrofluoride was added. The mixture was then shaken at 55°C for 1 hour to deprotect the 2'-hydroxyl groups of the target oligonucleotide. To this liquid, acetonitrile was added to precipitate the target oligonucleotide, and the resulting precipitate was then dissolved in water to prepare a crude liquid of the target oligonucleotide.

The amounts of the target oligonucleotide (FLP) and by-products were measured for the crude liquid of each of the oligonucleotides produced in Examples 13 to 16, and the results are shown in Table 3.

**[Table 3]**

| | THF (vol.%) | Liquid amount (mL/mmol) | Concentration of organic acid metal salt (mM) | Reaction temperature (°C) | Reaction time (hr) | FLP (%) | By-products (%) | Degradation products and the like (%) | Yield (%) |
|---|---|---|---|---|---|---|---|---|---|
| Example 13 | 33 | 59 | 20 | 35 | 6 | 69 | 0.8 | 18 | 64 |
| Example 14 | 37 | 198 | 72 | 45 | 4 | 66 | ≤ 0.2 | 21 | 67 |
| Example 15 | 50 | 30 | 55 | 55 | 4 | 72 | 0.3 | 16 | 67 |
| Example 16 | 53 | 33 | 13 | 35 | 8 | 75 | 0.8 | 13 | 64 |

When the composition of the reaction liquid (the type and volume % of the organic solvent, the type and concentration of the organic acid metal salt, and the like) changed, the reaction rate changed. As can be seen from Table 3, the reaction rate was increased by increasing the reaction time or the reaction temperature.

### Example 17

A crude liquid of the target oligonucleotide was obtained by performing the same operation under the same conditions as in Example 1, except that the target oligonucleotide (FLP) synthesized in Example 1 was changed to the oligonucleotide "5'-aC(F)uG(F)uU(O)auA(O)cU(F)caG(O)aA(F)ucgC(O)g-3' (SEQ ID NO: 2: 21mer, each nucleoside unit with (F) indicated on the right side of the base has a structure identical to that of 2'-F, each nucleoside unit with (O) indicated on the right side of the base has a structure identical to that of 2'-OMe, and other nucleoside units indicated by lowercase letters represent structural units identical to those of RNA)", and that "shaken at 35°C for 6 hours" was changed to "shaken at 35°C for 2 hours".

The amounts of the target oligonucleotide (FLP), by-products, and 2'-F-derived degradation products were measured for the crude liquid of each oligonucleotide produced in Example 17, and the results are shown in Table 4.

**[Table 4]**

| | FLP (%) | By-products (%) | Degradation products and the like (%) | Yield (%) | 2'-F-derived degradation products (%) |
|---|---|---|---|---|---|
| Example 17 | 76 | ≤ 0.2 | 13 | 74 | ≤ 0.1 |

In the case of 2'-F, a specific degradation product in which F is converted to a hydroxyl group or the like is likely to be generated under severe cleavage conditions. However, as shown in Table 4, even in the case of the oligonucleotide containing 2'-F and 2'-OMe, the oligonucleotide was efficiently cleaved by suppressing the generation of by-products, degradation products, and the like, and the oligonucleotides was produced with high purity and yield, as in Example 1.

### Example 18

The oligonucleotide "5'-G^A(L)C(F)U(F)G(O)U(F)U(F)A(L)U(F)A(M)C(F)U(F)C(F)A(M) G(O)A(M)A(M)U(F)C(F)G(O)C(F)G(O)-3' (SEQ ID NO: 3: 22mer, each nucleoside unit with (F) indicated on the right side of the base has a structure identical to that of 2'-F, each nucleosi de unit with (O) indicated on the right side of the base has a structure identical to that of 2'-O Me, each nucleoside unit with (M) indicated on the right side of the base has a structure identic al to that of 2'-MOE, each nucleoside unit with (L) indicated on the right side of the base has a structure identical to that of LNA, and other nucleoside units have structures identical to those of DNA, and ^ represents a phosphorothioate bond)" was synthesized in the same manner as in Example 1. To a mixed solution of 40 wt.% aqueous methyl amine solution/THF (volume ratio 75/25) having a liquid amount of 200 mL per mmol of the loading on the solid-phase carrier, s odium acetate was added so as to achieve a concentration of 150 mM. In this solution, a solid-phase carrier to which the synthesized target oligonucleotide was attached was immersed and sh aken at 21°C for 4 hours to cleave the target oligonucleotide from the solid-phase carrier and to deprotect the protecting groups (acetyl group (cytosine), benzoyl group (adenine), and isobutyry 1 group (guanine)) of the amino group (-NH₂) on the base moieties of the target oligonucleotide, and to remove the linker. Thereafter, the solid-phase carrier was removed by filtration and was hed with water to prepare a crude liquid of the target oligonucleotide.

The solid-phase carrier and the linker used are the same as those used in Example 1.

The amounts of the target oligonucleotide (FLP) and by-products were measured for the crude liquid of each oligonucleotide produced in Example 18, and the results are shown in Table 5.

**[Table 5]**

| | FLP (%) | By-products (%) | Degradation products and the like (%) | Yield (%) | 2'-F-derived degradation products (%) |
|---|---|---|---|---|---|
| Example 18 | 63 | ≤ 0.2 | 29 | 66 | 1.6 |

Even in the case of the sequence containing 2'-F at 50%, the oligonucleotide was efficiently cleaved by suppressing the generation of by-products, degradation products, and the like, and the oligonucleotide was produced with high purity and yield.

### Comparative Example 3

A crude liquid of an oligonucleotide of Comparative Example 3 was obtained by performing the same operation under the same conditions as in Example 1, except that the organic solvent used in Example 1 was changed to ethanol.

### Comparative Example 4

An experiment was performed in accordance with Example 1 of Patent Literature 1 using a solid-phase carrier to which the target oligonucleotide synthesized in Example 1 was attached (i.e., a solid-phase carrier before the cleavage step and the deprotection step).

In a mixed solution of 40 wt.% aqueous methylamine solution/2-propanol (1:1) having an amount of 100 mL per mmol of the loading on the solid-phase carrier, the solid-phase carrier to which the target oligonucleotide synthesized in Example 1 was attached was immersed and shaken at 25°C for 30 minutes to cleave the bond between the solid-phase carrier and the cleavable linker. Thereafter, the solid-phase carrier was removed by filtration and washed with a mixed solution of 40 wt.% aqueous methylamine solution/2-propanol (volume ratio 1:1). To the combined washing and filtrate, sodium bromide was added so as to achieve a concentration of 20 mM. The mixture was then shaken at 45°C for 4 hours to deprotect the protecting groups on the base moieties of the target oligonucleotide and to remove the linker. To this liquid, dimethyl sulfoxide and triethylamine trihydrofluoride were added, and the mixture was shaken at 55°C for 1 hour to deprotect the 2'-hydroxyl groups of the target oligonucleotide. To this liquid, acetonitrile was added to precipitate the target oligonucleotide, and the resulting precipitate was then dissolved in water to prepare a crude liquid of the oligonucleotide of Comparative Example 4.

### Comparative Example 5

An experiment was performed in accordance with Example 21 of Patent Literature 1 using a solid-phase carrier to which the target oligonucleotide synthesized in Example 1 was attached (i.e., the solid-phase carrier before the cleavage step and the deprotection step).

Specifically, the same operation was performed under the same conditions as in Comparative Example 4, except that the volume % of the solvent used in Comparative Example 4 was changed to 20 vol.%, the reaction temperature was changed to 25°C, and the reaction time was changed to 21 hours. Thus, a crude liquid of the oligonucleotide of Comparative Example 5 was obtained.

The amounts of the target oligonucleotide (FLP), by-products, degradation products, and the like in the crude liquid of each of the oligonucleotides produced in Comparative Examples 3 to 5 were measured, and the results are shown in Table 6 together with the measurement results of Example 1 described above.

**[Table 6]**

| | Organic solvent | Organic solvent vol.% | Additive | Reaction temperature (°C) | Reaction time (hr) | FLP (%) | By-products (%) | Degradation products and the like (%) | Yield (%) |
|---|---|---|---|---|---|---|---|---|---|
| Example 1 (Reiterated) | THF | 70 | Sodium acetate | 35 | 6 | 73 | ≤ 0.2 | 15 | 66 |
| Comparative Example 3 | Ethanol | 70 | Sodium acetate | 35 | 6 | 66 | ≤ 0.2 | 15 | 59 |
| Comparative Example 4 (Example 1 of Patent Literature 1) | 2-Propanol | 50 | Sodium bromide | 45 | 4 | 69 | 1.1 | 18 | 65 |
| Comparative Example 5 (Example 21 of Patent Literature 1) | 2-Propanol | 20 | Sodium bromide | 25 | 21 | 60 | ≤ 0.2 | 30 | 62 |

As shown in Table 6, in Comparative Example 3 in which the organic solvent was ethanol, the amount of the target oligonucleotide (FLP) was decreased, although the reaction was performed under the same reaction conditions as in Example 1 in which the organic solvent was THF. The reason for this is that the reaction rate for deprotection of the protecting groups on the base moieties (isobutyryl protecting group on guanine base) was low in an ethanol solvent, and thus a considerable amount of non-deprotected oligonucleotide remained. When the reaction conditions are made severe by increasing the reaction temperature or extending the reaction time in order to promote the deprotection, it is expected that FLP is degraded and the amount of by-products increases.

In Comparative Example 4 (Example 1 of Patent Literature 1), the amount of by-products (Unylinker-attached products) was large, the amount of degradation products and the like was large, and the amount of the target oligonucleotide (FLP) was decreased, as compared with Example 1 of the present invention. In Comparative Example 5 (Example 21 of Patent Literature 1), the amount of the degradation products and the like was very large and the amount of the target oligonucleotide (FLP) was decreased, as compared with Example 1 of the present invention.

As described above, according to the present invention, unlike Patent Literature 1, an organic solvent selected from an ether, a cyclic amide, and a cyclic urea is used, and an organic acid metal salt is further added. Therefore, an oligonucleotide can be efficiently cleaved by suppressing the generation of by-products, degradation products, and the like, and thus an oligonucleotide can be produced with high purity and yield.

The embodiments disclosed herein are to be considered in all respects as illustrative and not restrictive. The scope of the present invention is indicated by the entirety of the statement of the description, including the description of the above embodiments, and the scope of claims, and is intended to include all modifications within the meaning and scope equivalent to the scope of claims.

### Industrial Applicability

According to the production method of the present invention, an oligonucleotide can be cleaved with high purity and yield from a conjugate containing the oligonucleotide, a cleavable linker, and a support by suppressing the generation of cleavable-linker-attached by-products and degradation products.

## Claims

1. A method for producing an oligonucleotide, the method comprising:
cleaving an oligonucleotide from a conjugate containing the oligonucleotide, a cleavable linker, and a support in a solution containing an alkylamine and an organic solvent; and
optionally deprotecting a protecting group of the oligonucleotide, wherein:
the cleavable linker has a cyclic vicinal diol structure; and
the organic solvent is selected from an ether, a cyclic amide, and a cyclic urea.

2. The method according to claim 1, wherein the solution further contains an organic acid metal salt.

3. The method according to claim 2, wherein the organic acid is selected from a substituted or unsubstituted alkylsulfonic acid, a substituted or unsubstituted arylsulfonic acid, a substituted or unsubstituted alkanoic acid, and a substituted or unsubstituted alkanedioic acid.

4. The method according to claim 2 or 3, wherein the metal salt is an alkali metal salt.

5. A method for producing an oligonucleotide, the method comprising:
cleaving an oligonucleotide from a conjugate containing the oligonucleotide, a cleavable linker, and a support in a solution containing an alkylamine; and
optionally deprotecting a protecting group of the oligonucleotide, wherein:
the cleavable linker has a cyclic vicinal diol structure; and
the solution further contains an organic acid metal salt.

6. The method according to claim 5, wherein the organic acid is selected from a substituted or unsubstituted alkylsulfonic acid, a substituted or unsubstituted arylsulfonic acid, a substituted or unsubstituted alkanoic acid, and a substituted or unsubstituted alkanedioic acid.

7. The method according to claim 5 or 6, wherein the metal salt is an alkali metal salt.
